# EUROPÄISCHE PATENTSCHRIFT

(11) **EP 0 600 835 B1**
(45) Veröffentlichungstag und Bekanntmachung des Hinweises auf die Patenterteilung: **11.09.1996**
(21) Anmeldenummer: 93810809.9
(22) Anmeldetag: 23.11.1993
(51) Int. Cl.: C07C 239/20

(54) **Verfahren zur Herstellung von Phenylessigsäureester-Derivaten**
Method for the production of derivatives of phenylacetic acid esters
Procédé pour la fabrication de dérivés d'esters de l'acide phénylacétique

(30) Priorität: 01.12.1992 CH 3683/92
(43) Veröffentlichungstag der Anmeldung: 08.06.1994
(73) Patentinhaber: CIBA-GEIGY AG, 4002 Basel (CH)
(72) Erfinder: Pfiffner, Albert, Dr., CH-8180 Bülach (CH); Trah, Stephan, Dr., D-79110 Freiburg (DE); Ziegler, Hugo, Dr., CH-4108 Witterswil (CH)

(56) Entgegenhaltungen:
- EP-A- 0 460 575
- EP-A- 0 463 488
- WO-A-92/18487
- WO-A-92/18494
- CH-A- 403 791
- DE-B- 1 276 046
- DE-B- 2 137 538

## Beschreibung

Die vorliegende Erfindung betrifft ein 2-Aminooxymethylenphenylessigsäureester-Derivat der Formel I 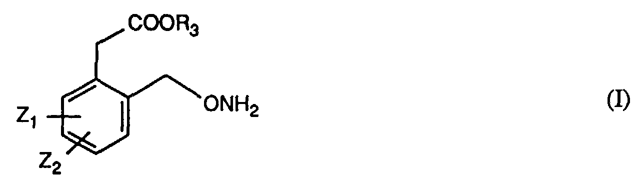 als freie Base und als Salz mit Proton-Säuren, seine Herstellung sowie seine Verwendung zur Gewinnung von im Pflanzenschutz wertvollen Mikrobiziden der Formel VI.

In der obengenannten Formel bedeuten
Z₁, Z₂ Wasserstoff, Halogen, Hydroxy, C₁-C₄-Alkyl, C₁-C₄-Alkoxy, C₁-C₄-Halogenalkyl,
C₁-C₄-Halogenalkoxy, C₁-C₄-Alkylcarbonyl, C₁-C₄-Alkoxycarbonyl, Phenoxy, Nitro, Cyano,
Z₁ und Z₂ zusammen mit dem Phenylrest, an den sie gebunden sind, ein gegebenenfalls hydriertes Naphthalin, und
R₃ Wasserstoff oder C₁-C₁₂-Alkyl.

Als Salze kommen solche mit Säuren HY in Frage, wobei Y das Säureanion ist, z.B. Chlorwasserstoff, Bromwasserstoff, Jodwasserstoff, Perchlorsäure, gegebenenfalls substituierte Benzolsulfonsäure, Methansulfonsäure, Trifluormethansulfonsäure, Tetrafluorborsäure, Hexafluorphosphorsäure, Phosphorsäure, Schwefelsäure, Schweflige Säure, Carbonsäuren allgemein (z.B. Ameisensäure, Essigsäure, Trifluoressigsäure etc.). Bevorzugt sind Mineralsäuren, besonders Chlorwasserstoff und Schwefelsäure.

Das erfindungsgemässe Verfahren besteht im Schritt A aus der Reaktion eines substituierten o-Tolylessigsäureesters II mit einem Benzoylhydroxylamin III zu benzoylierten 2-Aminooxymethylenphenylessigsäureestern IV und im Schritt B aus der sauren Verseifung(= Hydrolyse) zum Endprodukt I gemäss

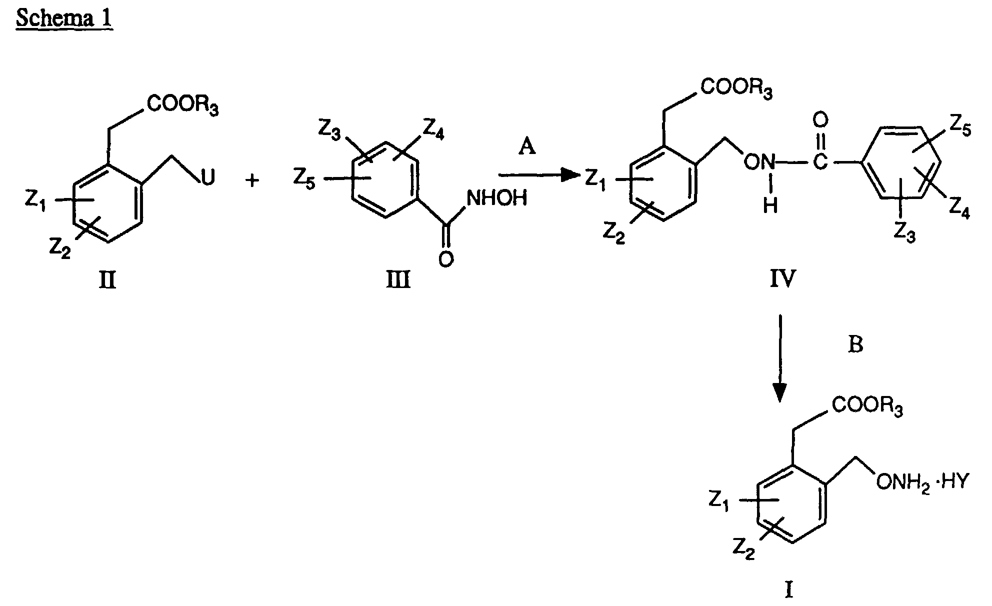

Darin bedeutet U eine nucleofuge Abgangsgruppe,
R₃, Z₁ und Z₂ haben die oben angegebenen Bedeutungen,
Z₃, Z₄ und Z₅ sind gleich oder verschieden und bedeuten Wasserstoff, gegebenenfalls verzweigtes C₁-C₆-Alkyl, Halogen, Hydroxy, gegebenenfalls verzweigtes C₁-C₆-Alkoxy, Nitro, Cyano, N(R₅)₂, SO₂NH₂, gegebenenfalls substituiertes Phenoxy, CF₃, Mercapto, wobei die beiden
R₅ gleich oder verschieden sind und Wasserstoff und/oder gegebenenfalls verzweigtes C₁-C₆-Alkyl bedeuten,
und worin auch
Z₃ und Z₄ in benachbarter Stellung zusammen eine Methylendioxy-Brücke bilden oder mit dem Phenylring, an den sie gebunden sind, einen Naphthylring bilden können.

Bevorzugte Substituenten Z₃, Z₄ und Z₅ sind Wasserstoff, Methyl und Halogen, besonders bevorzugt ist Wasserstoff. R₃ ist bevorzugt Wasserstoff oder C₁-C₄-Alkyl, besonders bevorzugt Methyl.

Als nucleofuge Abgangsgruppe U kommen z.B. Halogenide wie Chlorid, Bromid, Jodid in Frage, ferner Benzolsulfonat, Toluolsulfonat, Methansulfonat, Triflat oder Acetat. Besonders bevorzugt ist Bromid.

Zweckmässig werden in beiden Teilschritten A und B Verdünnungsmittel eingesetzt und die Reaktionen im Temperaturbereich von 0°C bis zum Siedepunkt des Gemisches durchgeführt.

Im Teilschritt A wird zweckmässig eine anorganische oder organische Base bzw. ein Basengemisch zugesetzt, während die Hydrolyse im Teilschritt B unter sauren Bedingungen erfolgt.

Das neue erfindungsgemässe Verfahren zeigt in beiden Teilschritten A und B leichte Zugänglichkeit der Reaktionspartner, glatte präparative Reaktion und überraschend hohe Ausbeuten, selbst in den Fällen, wo das Ausgangsprodukt der Formel II als unreines Rohprodukt eingesetzt wird. Es stellt daher einen ganz wesentlichen Beitrag zur vereinfachten Gewinnung von mikrobiziden 2-Phenylacrylaten, 2-Phenylalkoxyacrylaten und Phenylmethoxyiminoacetaten dar, wie sie etwa in der WO 90/07493, EP-A-370 629, EP-A-414 153, EP-A-426 460, EP-A-460 575, EP-A-463 488, EP-A-472 300, WO 92/18494, WO 92/18487 u.a. beschrieben sind.

Die so gewonnenen Verbindungen der Formel I lassen sich, sei es als Basen oder als Salze, durch Reaktion mit Aldehyden oder Ketonen zu entsprechenden Aldimino- bzw. Ketimino-Derivaten der Formel V umsetzen, aus denen man in üblicher Art durch Weiterreaktion in der Acetat-Seitenkette zu den in der vorstehend genannten Literatur beschriebenen Mikrobiziden kommt (Reaktionsfolgen C und D):

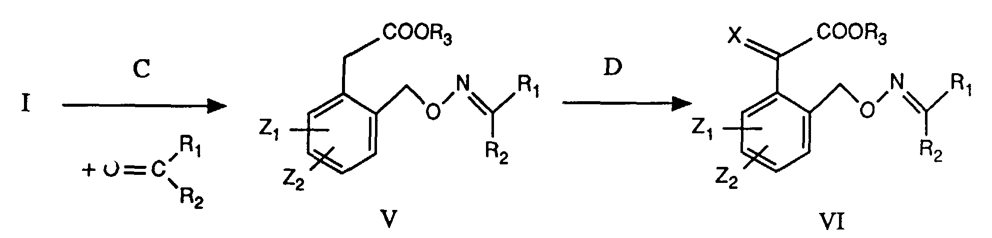

In der letzen Formel VI bedeutet X CHO(C₁-C₄-Alkyl), NO(C₁-C₄-Alkyl), oder unsubstituiertes oder anderweitig substituiertes CH₂;
R₁ und R₂ können gleich oder verschieden sein und bedeuten Wasserstoff, Cyano, gegebenenfalls verzweigtes C₁-C₁₀-Alkyl, C₁-C₄-Halogenalkyl, C₃-C₆-Cycloalkyl, C₃-C₆-Halogencycloalkyl, C₃-C₆-Cycloalkyl-C₁-C₄-alkyl, C₁-C₄-Alkoxy-C₁-C₄-alkyl, C₁-C₄-Alkylthio-C₁-C₄-alkyl, Arylthio-C₁-C₄-alkyl, C₂-C₆-Alkenyl, C₂-C₅-Halogenalkenyl, C₃-C₆-Cycloalkenyl, C₃-C₆-Halogencycloalkenyl, C₂-C₆-Alkinyl, C₁-C₆-Alkoxy, C₁-C₆-Halogenalkoxy, C₁-C₄-Alkylthio, Benzylthio, C₁-C₄-Alkylcarbonyl, gegebenenfalls substituiertes Phenylcarbonyl, gegebenenfalls substituiertes Benzylcarbonyl, C₁-C₄-Alkoxycarbonyl, gegebenenfalls substituiertes Phenoxycarbonyl, gegebenenfalls substituiertes Benzyloxycarbonyl, gegebenenfalls substituiertes Aryl, gegebenenfalls substituiertes Aryloxy, gegebenenfalls substituiertes Arylthio, gegebenenfalls substituiertes Aryl-C₁-C₄-alkyl, gegebenenfalls substituiertes Aryl-C₁-C₄-alkenyl, gegebenenfalls substituiertes Aryloxy-C₁-C₄-alkyl, gegebenenfalls substituiertes Arylthio-C₁-C₄-alkyl, gegebenenfalls substituiertes Heteroaryl, gegebenenfalls substituiertes Heteroaryloxy, gegebenenfalls substituiertes Heteroarylthio, gegebenenfalls substituiertes Heteroaryl-C₁-C₄-alkyl, gegebenenfalls substituiertes Heteroaryl-C₂-C₄-alkenyl, gegebenenfalls substituiertes Heteroaryloxy-C₁-C₄-alkyl, gegebenenfalls substituiertes Heterocyclyl, gegebenenfalls substituiertes Heterocyclyloxy, gegebenenfalls substituiertes Heterocyclylalkyl, N(R₄)₂, wobei die Bedeutungen von R₄ gleich oder verschieden sind und H, C₁-C₆-alkyl, gegebenenfalls substituiertes Phenyl bedeuten, oder beide R₄ bilden zusammen mit dem Stickstoffatom, an das sie gebunden sind, einen 5- bis 7-gliedrigen, gegebenenfalls substituierten Ring, der Heteroatome aufweisen kann; oder R₁ und R₂ bedeuten
-CON(R₄)₂, Nitro, Halogen, -S(O)ₙR₄, worin n 0, 1 oder 2, ist oder (CH₂)ₘPO(OR₄)₂, worin m 0 oder 1 ist, oder R₁ und R₂ zusammen bilden einen carbocyclischen oder heterocyclischen Ring, der gegebenenfalls substituiert sein kann,
während R₃, Z₁ und Z₂ die für Formel I genannte Bedeutung haben.

Die Erfindung bezieht sich auch auf die Herstellung von Verbindungen der Formel VI gemäss den Reaktionsschritten A, B, C und D über die Zwischenprodukte der Formeln IV, I und V.

Demgemäss bezieht sich die Erfindung auch auf ein Verfahren zur Herstellung der vorstehend definierten Verbindungen der Formel VI, das gekennzeichnet ist durch
A) Reaktion eines substituierten o-Tolylessigsäureesters der Formel II 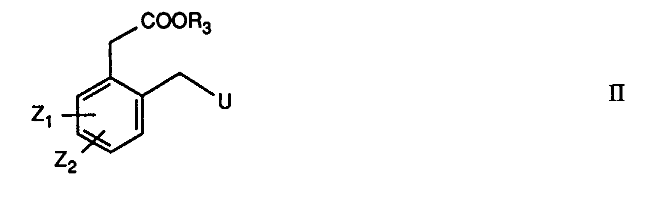 mit einem Benzoylhydroxylamin III 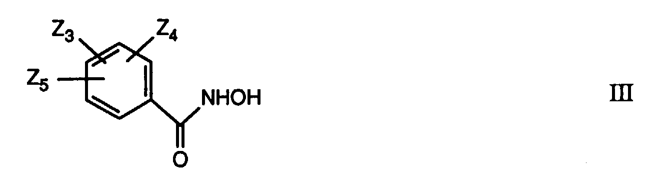
B) saure Verseifung des so gewonnenen benzoylierten 2-Aminooxymethylenphenylessigsäureesters der Formel IV 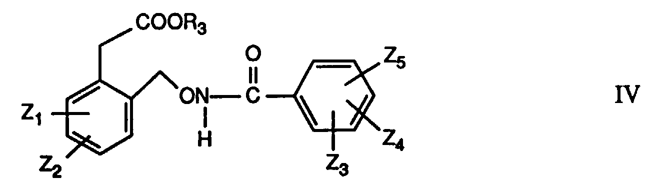 mit einer Säure HY,
C) Reaktion des 2-Aminooxymethylenphenylessigsäureesters der Formel I 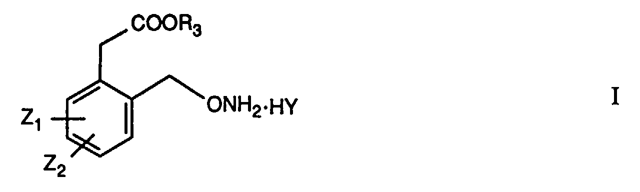 mit einem Keton R₁-CO-R₂ und
D) Ueberführung der Acetat-Seitenkette des gebildeten Zwischenprodukts V 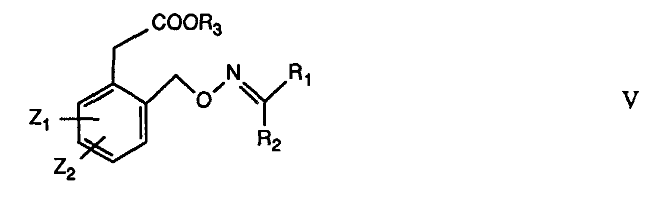 in die Seitenkette 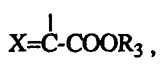 worin X wahlweise C₁-C₄-Alkyl-O-CH oder C₁-C₄-Alkyl-O-N bedeutet, wobei Z₁ bis Z₅ und R₁ bis R₃ sowie U die für die weiter oben genannten Formeln I bis V angegebenen Bedeutungen haben, während Y ein Säureanion ist.

Zur Gewinnung von Verbindungen der Formel VI wurden in der Literatur bisher folgende umständliche Reaktionsfolgen vorgeschlagen; bei denen hohe Ausbeuteverluste zu verzeichnen sind (unvollständige Reaktion; Nebenreaktionen).

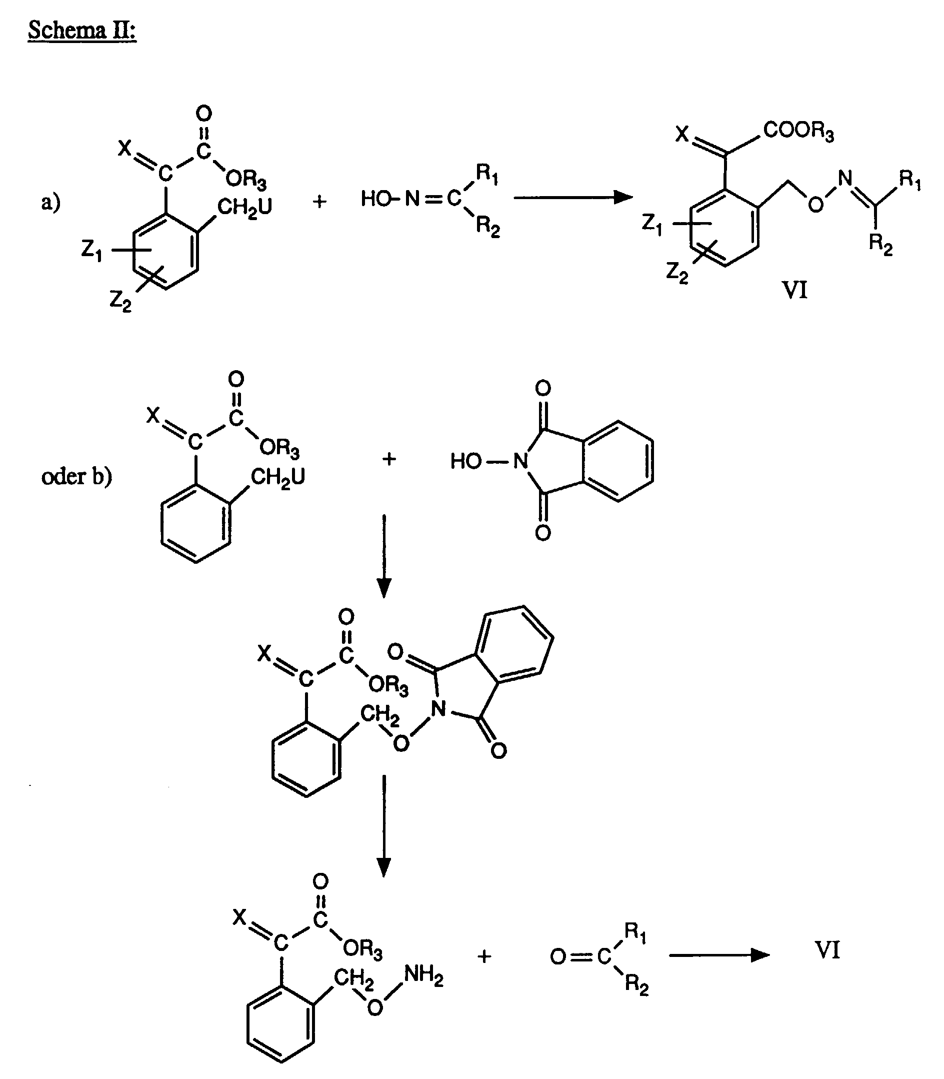

Demgegenüber stellt das Verfahren gemäss vorliegender Erfindung eine bedeutende Bereicherung der Technik dar.

Im folgenden werden für die Schritte A, B, C und D Erläuterungen und präparative Beispiele gebracht.

### Reaktionssequenz A:

Die Verbindungen der allgemeinen Formel II und III, worin Z₁ bis Z₅, R₃ und U die oben angegebenen Bedeutungen haben, werden vorteilhaft in Gegenwart einer Base, zur Reaktion gebracht. Als anorganische oder organische Base dienen beispielsweise Alkalioder Erdalkaliverbindungen, z.B. die Hydroxide, Oxide oder Karbonate von Lithium, Natrium, Kalium, Magnesium, Calcium, Strontium und Barium oder auch Hydride wie z.B. Natriumhydrid, ferner Oxide wie ZnO, Al₂O₃, Ag₂O etc. Als organische Basen seien beispielsweise NH₃, Alkylamin, Dialkylamin, Piperidin, Chinolin, tertiäre Amine wie Triethylamin, Triethylendiamin, Pyridin, 4-Dimethylaminopyridin genannt, ferner Alkoholate wie NaOCH₃, NaOEt, K-tert. Butylat etc.

Obgleich nicht zwingend notwendig, lassen sich zur Reaktion Lösungs- bzw. Verdünnungsmittel vorteilhaft verwenden. Es können z.B. genannt werden: Halogenkohlenwasserstoffe, insbesondere Chlorkohlenwasserstoffe, wie Tetrachlorethylen, Tetrachlorethan, Dichlorpropan, Dichlormethan, Dichlorbutan, Chloroform, Tetrachlorkohlenstoff, Trichlorethan, Trichlorethylen, Pentachlorethan, 1,2-Dichlorethan, 1,1-Dichlorethan, 1,2-cis-Dichlorethylen, Chlorbenzol, Fluorbenzol, Brombenzol, Dichlorbenzol, Dibrombenzol, Chlortoluol, Trichlortoluol; Ether, wie Ethylpropylether, Methyl-tert.-butylether, n-Butylethylether, Di-n-butylether, Di-isobutylether, Diisoamylether, Diisopropylether, Anisol, Cyclohexylmethylether, Diethylether, Ethylenglykoldimethylether, Tetrahydrofuran, Dioxan, Thioanisol, Dichlordiethylether; Nitrokohlenwasserstoffe wie Nitromethan, Nitroethan, Nitrobenzol, Chlornitrobenzol, o-Nitrotoluol; Nitrile wie Acetonitril, Butyronitril, Isobutyronitril, Benzonitril, m-Chlorbenzonitril; aliphatische, aromatische oder cycloaliphatische Kohlenwasserstoffe, wie Heptan, Hexan, Octan, Nonan, Cymol, Benzinfraktionen innerhalb eines Siedepunktintervalles von 70°C bis 190°C, Benzol, Toluol, Cyclohexan, Methylcyclohexan, Dekalin, Petrolether, Ligroin, Trimethylpentan, wie 2,3,3-Trimethylpentan; Ester wie Ethylacetat, Isobutylacetat; Amide z.B. Formamid, N-Methylformamid, N-Methylpyrrolidon, Dimethylformamid; Ketone, wie Aceton, Methylethylketon; Dimethylsulfoxid. Auch Gemische der genannten Lösungs- und Verdünnungsmittel kommen in Betracht.

Die Reaktionstemperaturen liegen bevorzugt im Bereich von 0°C bis zur Siedetemperatur des Verdünnungsmittels.

Besonders bevorzugt sind als Basen im Reaktionsschritt A Alkalicarbonate, vor allem Natriumcarbonat oder Kaliumcarbonat und als eines der bevorzugten Verdünnungsmittel Acetonitril, insbesondere im Temperaturbereich von 60°C bis 65°C.

Die Isolierung der so hergestellten Verbindung der Formel IV kann nach an sich bekannten Methoden erfolgen, z.B. durch Abdampfen des Verdünnungsmittels und Aufnahme des Rückstandes in Ethylacetat, das dann mit wässriger Kaliumcarbonat-Lösung gewaschen wird. Nach dem Trocknen über einem Entwässerungsmittel wie Natriumsulfat wird zur Trockne eingedampft. Das so erhaltene Hydroxamat der Formel IV, und das ist ein wichtiger Vorteil des vorliegenden erfinderischen Verfahrens, kann ohne weitere Reinigung direkt weiter verwendet werden.

Die Ausgangsprodukte der Formeln II und III sind entweder bekannt oder können nach bekannten Methoden hergestellt werden (siehe z.B. Org. Synth. Coll. Vol. II, 67 und J. Am. Chem. Soc. 31, 3759 (1966)).

### Reaktionssequenz B:

Das unter Sequenz A erhaltene Rohprodukt der Formel IV, worin Z₁-Z₅ und R₃ die oben angegebenen Bedeutungen besitzen, wird in einem Verdünnungsmittel, welches vorzugsweise ein Alkohol der Formel R₃-OH ist, der zusätzlich mit einem weiteren wie unter A) genannten Lösungsmittel versetzt sein kann, in Gegenwart einer Säure der Formel HY, wobei Y das Anion der Säure ist, bei einer Reaktionstemperatur von 0°C bis zum Siedepunkt des Verdünnungsmittels, vorzugsweise zwischen 20°C und der Rückflusstemperatur des Verdünnungsmittels, zur Verbindung der allgemeinen Formel I verseift, wobei Z₁, Z₂, R₃ und Y die angegebenen Bedeutungen haben.

Als Säuren HY kommen vorteilhaft Halogenwasserstoffsäure (wie Chlorwasserstoff-, Bromwasserstoff-, Jodwasserstoffsäure), Sulfonsäure (wie gegebenenfalls substituierte Benzolsulfonsäure, Methansulfonsäure, Trifluormethansulfonsäure), Carbonsäure (wie Ameisensäure, Essigsäure, Trifluoressigsäure), Perchlorsäure, Tetrafluorborsäure, Phosphorsäure, Hexafluorphosphorsäure, Schwefelsäure oder schweflige Säure in Frage. Diese Aufzählung ist nicht limitierend für andere mögliche Säuren.

### Reaktionssequenz C:

Das unter Sequenz B erhaltene Salz der Formel I lässt sich mit einem Keton der Formel VII 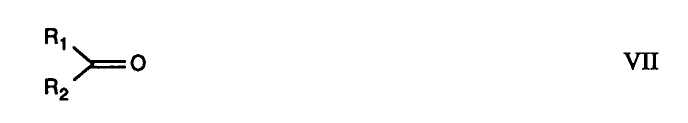 worin R₁ und R₂ die vorgängig erwähnten Bedeutungen besitzen, in einem inerten Verdünnungsmittel, wie einem Ether (z.B. Tetrahydrofuran oder Dioxan oder Dimethoxyether), einem Ester (z.B. Ethylacetat), einem halogenierten Kohlenwasserstoff (z.B. 1,2-Dichlorethan oder Chloroform oder Methylenchlorid), einem Amid (z.B. Dimethylformamid oder N-Methylpyrrolidon), einem Aromaten (z.B. Benzol oder Toluol), Acetonitril, Dimethylsulfoxid, in Gegenwart einer Base, wie sie unter der Sequenz A definiert ist, zur Reaktion bringen. Bevorzugt wird die Umsetzung in Pyridin, das zugleich als Verdünnungsmittel und Base dient. Die Reaktion findet im Temperaturbereich von 0°C bis zur Siedetemperatur des Verdünnungsmittels resp. der Base statt. Bevorzugt sind Temperaturen zwischen 50°C und der Rückflusstemperatur des Verdünnungsmittels. Wird die Umsetzung in Pyridin vorgenommen, sind Temperaturen zwischen 80°C und 116°C besonders bevorzugt.

Die Isolierung und Reinigung der Produkte der allgemeinen Formel V kann nach bekannten Methoden erfolgen, indem z.B. das Verdünnungsmittel abdestilliert und der verbleibende Rückstand in Wasser aufgenommen wird. Nach dem Ansäuern wird extrahiert. Das so erhaltene Rohprodukt kann nach bekannten Methoden gereinigt werden, z.B. durch Destillation oder Chromatographie. Das Produkt kann als Isomerengemisch (E/Z) anfallen.

### Reaktionssequenz D:

a) In dem unter Sequenz C erhaltenen Produkt der Formel V lässt sich nach üblichen Methoden die Acetat-Seitenkette in eine Hydroxyacrylsäureester-Seitenkette verwandeln. Hierzu lässt man Methylformiat in Gegenwart einer starken Base wie Na-Alkoholat (besonders Na-Methylat) oder NaH, NaNH₂, K-tert.Butylat etc. auf das Zwischenprodukt der Formel V einwirken. Als Lösungsmittel sind inerte polare Vertreter wie Dimethylformamid, Ether (Tetrahydrofuran, Dioxan etc), Alkohole wie tert. Butanol oder Methanol und Ketone wie tert. Butylmethylketon besonders geeignet. Die Reaktionstemperatur beträgt -10°C bis +60°C, bevorzugt 0°C bis 40°C.
   Die nachfolgende Alkylierung zur Alkoxyacrylsäureester-Seitenkette kann mit z.B. Alkyljodid oder Diazomethan erfolgen. Geeigneterweise verwendet man zur Gewinnung des Methoxyacrylsäureesters Dimethylsulfat in basischem Medium mit Alkylcyanid als Lösungsmittel, bevorzugt Acetonitril. Die Reaktionstemperatur beträgt 0°C bis +70°C.
b) In dem unter Sequenz C erhaltenen Produkt der Formel V lässt sich die Acetat-Seitenkette mit Hilfe von Nitrit in basischem Milieu in die Hydroxyiminoglyoxylsäureester-Seitenkette überführen, die nachfolgend auf die gleiche unter a) genannte Art alkyliert werden kann. Zur Einführung der Hydroxyiminogruppe werden geeigneterweise Alkylnitrite wie Ethylnitrit, tert. Butylnitrit, Isopentylnitrit etc. in Gegenwart einer Base wie NaOH, KOH, NaH, Alkali-Alkoholat (wie K-tert. Butylat oder NaOCH₃) verwendet. Die Reaktionstemperatur beträgt -10°C bis +60°C, bevorzugt 0°C bis 40°C. Inerte polare Lösungsmittel lassen sich verwenden, beispielsweise Ether, Alkohole und Ketone, bevorzugt die unter a) aufgeführten Vertreter.

### Herstellungsbeispiele:

### Beispiel 1 (Reaktionssequenz A)

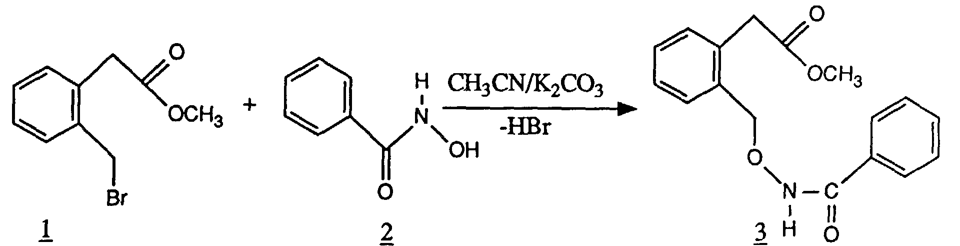

Ein Gemisch aus 102 g (0,74 Mol) Benzhydroxamsäure 2, 111 g (0,80 Mol) pulverisiertem Kaliumcarbonat und 1400 ml Acetonitril wird auf 60°C erwärmt. Nach 0,5 Std. werden 162 g o-Brommethylphenylessigsäuremethylester 1, der als 68%iges Rohprodukt aus der Bromierung des o-Tolylessigsäuremethylesters mit N-Bromsuccinimid vorliegt, gelöst in 100 ml Acetonitril, während 0,5 Std. bei 60°C-65°C zugetropft und 5,5 Std. bei dieser Temperatur nachgerührt.

Zur Aufarbeitung wird auf 25°C abgekühlt, durch Hyflo Super Cel filtriert und am Wasserstrahlvakuum eingedampft Das zurückbleibende Oel wird in 1000 ml Ethylacetat aufgenommen und mit 500 ml einer 6 %igen Kaliumcarbonat-Lösung gewaschen. Die Phasen werden getrennt und die wässrige Phase einmal mit 300 ml Ethylacetat nachextrahiert. Die vereinigten organischen Phasen werden einmal mit 200 ml Wasser gewaschen, über Natriumsulfat getrocknet, filtriert und zur Trockne eingedampft Man erhält 182 g des Benzhydroxamates 3 in Form eines orangen Oels. Eine weitere Reinigung findet nicht statt.

### Beispiel 2 (Reaktionssequenz B)

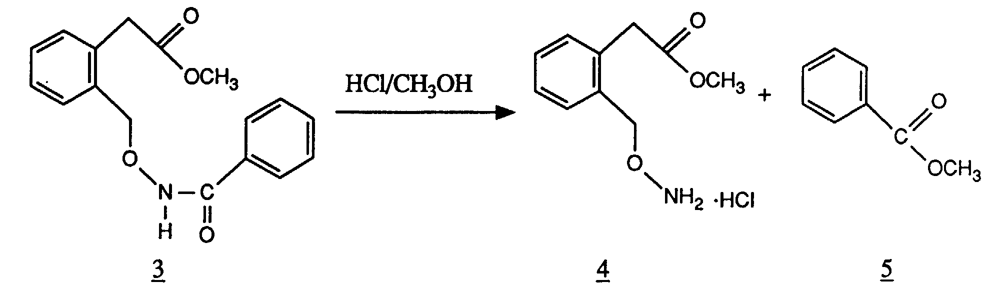

Eine Lösung aus 182 g ungereinigtem Benzhydroxamat 3, wie es im Beispiel 1 erhalten wurde, und 900 ml einer 8 %igen Chlorwasserstoffsäure in Methanol wird auf 60°C erwärmt. Nach 1 Std. wird das überschüssige Methanol abgedampft und der Rückstand, bestehend aus dem Amin-Hydrochlorid 4 und Benzoesäuremethylester 5 mit 500 ml Diethylether versetzt und 0,5 Std. bei 35°C gerührt. Das nun klebrig ausgefallene Amin-Hydrochlorid 4 wird nach Abkühlen auf 20°C von der Ether-Lösung abgetrennt und mit 500 ml Tetrahydrofuran bei 40°-50°C digeriert. Nach Abkühlen und Abnutschen erhält man 65.5 g (= 62 % d.Th. ausgehend vom Bromid 1) eines schwach hellgrünen, feinen Kristallpulvers mit Schmelzpunkt 157°-158°C (Zers.).

Die beschriebenen Reaktionssequenzen A und B zeichnen sich aus durch die Verwendung von einfach zugänglichen und billigen Ausgangsmaterialien und Reaktionshilfsmitteln. Ein entscheidender Vorteil gegenüber bisher bekannten Synthesemethoden zur Herstellung der Verbindungen der Formel VI liegt in der Möglichkeit, das aus der Bromierung von o-Tolylessigsäuremethylester mit N-Bromsuccinimid (NBS) entstehende Rohprodukt ungereinigt einzusetzen und trotzdem zu einer über beide Sequenzen A und B hohen Ausbeute zu 4 zu gelangen (ca.60-70 %).

Der zweite entscheidende Vorteil gegenüber bisher bekannten Verfahren zur Herstellung der Verbindung der Formel VI besteht darin, mit 4 eine Verbindung zur Verfügung zu haben, welche es erlaubt, auch mit jenen Ketonen 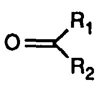 in vernünftigen Ausbeuten zu den Verbindungen der Formel VI zu gelangen, mit denen ein Umsatz als Oxim gemäss Schema II a) überhaupt nicht oder in nur unbefriedigenden Ausbeuten verläuft.

### Beispiel 3 (Reaktionssequenz C)

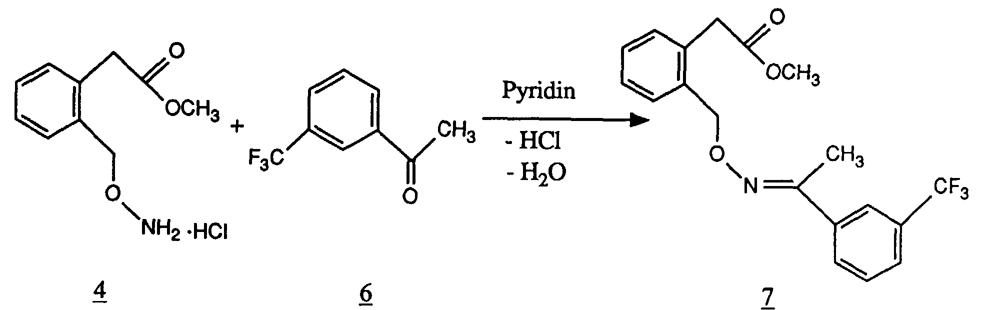

Zu einer Lösung von 53,1 g (0,282 Mol) 3-Trifluormethyl-acetophenon 6 in 530 ml Pyridin werden bei 20°C 65,4 g (0,282 Mol) Aminhydrochlorid 4 gegeben und auf 90°C erwärmt. Nach ca. 2 Std. ist die Reaktion beendet und das überschüssige Pyridin wird im Wasserstrahlvakuum abgedampft. Der Rückstand wird mit 600 ml kaltem Wasser versetzt, mit konzentrierter Chlorwasserstoffsäure aufpH 1-2 gestellt und dreimal mit Ethylacetat extrahiert. Die vereinigten organischen Phasen werden einmal mit Wasser und einmal mit 10 %iger Natriumcarbonat Lösung gewaschen, über Natriumsulfat getrocknet, filtriert und im Wasserstrahlvakuum zur Trockne eingedampft. Man erhält 101 g eines gelben Oels (=̂ 98 % d.Th.), welches nach NMR reines Oxim 7 als E/Z-Gemisch enthält (E: 87 %; Z: 13 %). Das Oxim kann auch im Feinvakuum destilliert werden.
Sdp. 168°-178°C/0.1 mbar.

### Beispiel 4: (Reaktionssequenz D)

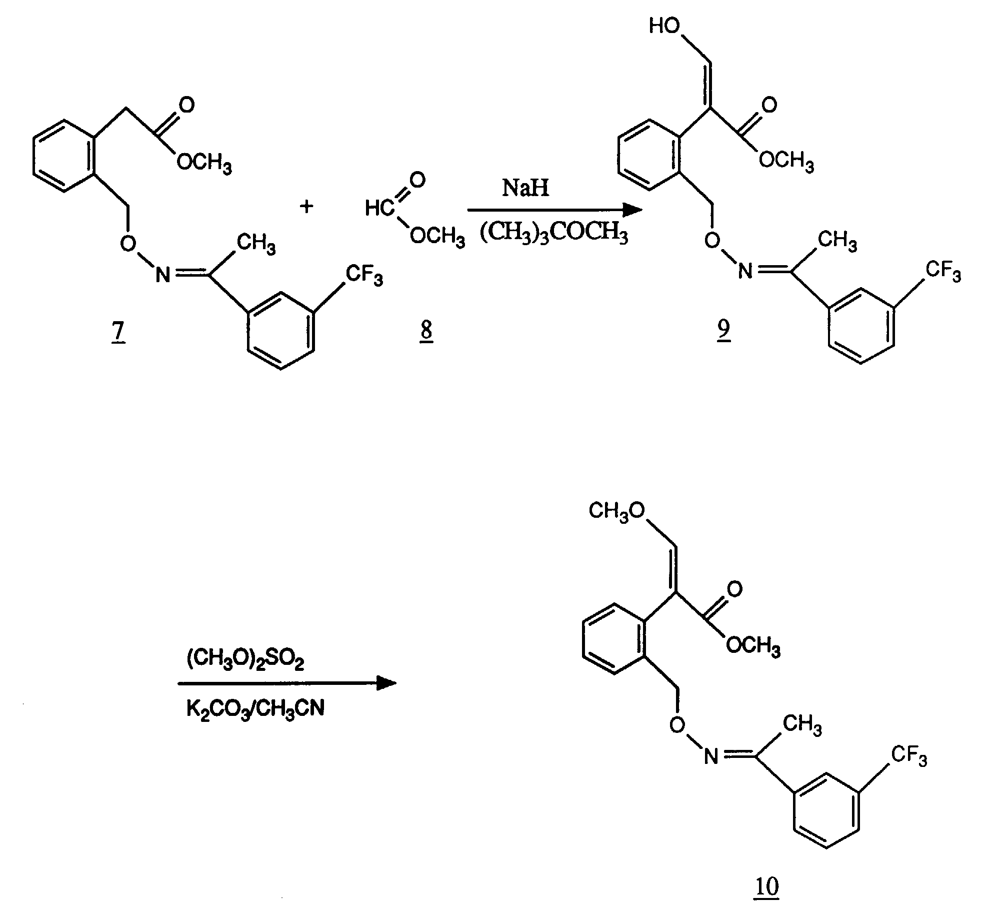

Zu einer Suspension von 6.7 g (0,28 Mol) Natriumhydrid in 100 ml tert.-Butyl-methylether wird nach Zusatz von 0,5 ml Methanol eine Mischung aus 46,0 g (0,125 Mol) Oximether 7, 31,3 g (0,52 Mol) Methylformiat und 150 ml tert.-Butyl-methylether bei 29°-35°C zugetropft. Zu Beginn muss eine kleine Induktionsperiode abgewartet werden. Die Oximether-Lösung wird innert 3 Stunden zugetropft. Anschliessend wird noch 5 Stunden bei 30°-35°C nachgerührt. Zur Aufarbeitung wird auf 0°-5°C gekühlt, ca. 2 ml Methanol und anschliessend 100 ml Wasser werden zugetropft, mit ca. 20 ml Essigsäure wird auf pH 5 gestellt, und die Phasen werden getrennt. Die wässrige Phase wird zweimal mit 300 ml tert.-Butyl-methylether nachextrahiert. Die vereinigten organischen Phasen werden zweimal mit 5 %iger Natriumcarbonat-Lösung gewaschen, über Natriumsulfat getrocknet, filtriert und zur Trockne eingedampft. Man erhält 48,3 g (≙ 98,1 % d.Th.) eines gelben Oels 9 welches nach NMR rein ist (E/Z-Gemisch).

Zu dem Gemisch aus 48,2 g (0,122 Mol) Enol 9 25,3 g (0,183 Mol) pulverisiertem Kaliumcarbonat in 500 ml Acetonitril werden bei 25°C 16,4 g (0,13 Mol) Dimethylsulfat innert 30 Minuten zugetropft und anschliessend noch 5 Std. bei 25°C nachgerührt. Zur Aufarbeitung wird das Acetonitril im Vakuum abgedampft und der Rückstand mit 500 ml Wasser und 500 ml Toluol versetut. Die Phasen werden getrennt und die wässrige Phase wird einmal mit 200 ml Toluol nachextrahiert. Die vereinigten organischen Phasen werden einmal mit Wasser gewaschen und im Vakuum zur Trockne eingedampft. Zuletzt wird bei 60°C und 0,13 mbar vollständig getrocknet. Man erhält 48,0 g (=̂ 96,5 % d.Th.) eines orangen Oels 10, welches nach NMR 89 % des E und anti; 8 % E und syn; und 3 % Z-Isomeres enthält.

### Beispiel 5 (Reaktionssequenz D)

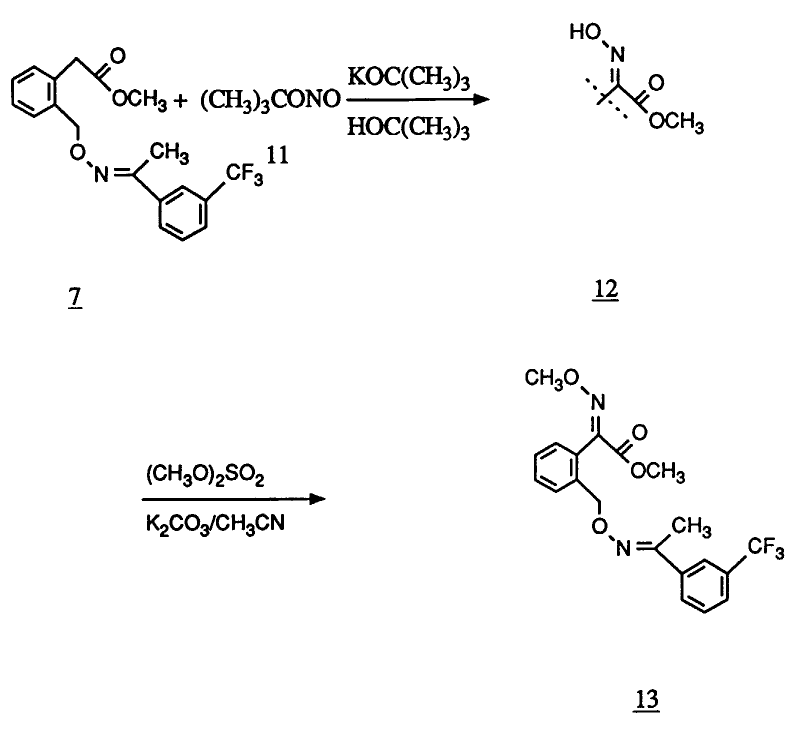

Eine Lösung aus 5,7 g (15,6 mMol) Oximether 7, 6,0 g (52,3 mMol) tert.-Butylnitrit (90 %) und 10 ml tert.-Butanol wird bei 30°C innert 1 Std. zu einer Lösung von 1,8 g (15,6 mMol) Kalium-tert.-butylat in 15 ml tert.-Butanol getropft. Danach wird 2 Stunden bei 35°C nachgerührt. Zur Aufarbeitung wird auf 10°C abgekühlt, 10 ml Wasser und anschliessend 1,2 g Essigsäure werden zugetropft. Das Gemisch wird nun in 200 ml Ethylacetat aufgenommen, mit Wasser gewaschen und im Wasserstrahlvakuum zur Trockne eingedampft. Man erhält 5,2 g eines orangen Oels 12, welches zwecks Reinigung in 25 ml Hexan erwärmt wird. Dabei beginnt das Produkt zu kristallisieren. Nach dem Abkühlen auf 20°C wird abgesaugt und das nun hellgelbe Kristallisat mit Schmelzpunkt von 132°-138°C noch aus 8 ml Toluol und 1 ml Hexan umkristallisiert. Daraus erhält man 2,4 g (=̂ 39 %) eines weissen Kristallpulvers mit Schmelzpunkt von 141-144°C.

Zu einem Gemisch aus 2,3 g (5,8 mMol) des vorgängig erhaltenen Oxims 12, 1,6 g (11,6 mMol) pulverisiertem Kaliumcarbonat und 25 ml Acetonitril werden bei 25°C 0,79 g (6,3 mMol) Dimethylsulfat gegeben. Nach 3 stündigem Nachrühren wird das Acetonitril im Wasserstrahlvakuum abgedampft, der Rückstand auf Wasser gegeben und zweimal mit Ethylacetat extrahiert. Die vereinigten organischen Phasen werden mit Wasser gewaschen, über Natriumsulfat getrocknet, filtriert und eingedampft Der Dioximether 13 wird durch Chromatographie an Kieselgel mit Ethylacetat/Hexan (1:2) isoliert. Man erhält hellgelbe Kristalle mit Schmelzpunkt 67°-69°C.

### Beispiel 6 (Reaktionssequenz C)

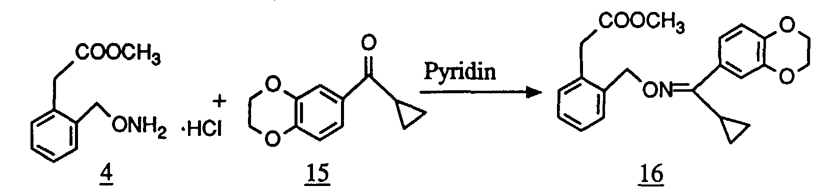

38,8 g (1,4-Benzodioxan-6-yl)-cyclopropylketon 15 und 44,0 g 4 werden in 400 ml Pyridin 4 Stunden bei 100°C gerührt. Anschliessend wird das Lösungsmittel mittels Destillation entfernt und der Rückstand mit 0,5 Liter Eiswasser und 1 Liter Diethylether verrührt. Die Wasserphase wird abgetrennt, die organische Phase mit Wasser gewaschen, getrocknet (Natriumsulfat) und im Vakuum eingeengt. Der Rückstand wird mit Dichlormethan an Kieselgel chromatographiert: 32 g 16 als hellgelbes Oel, MS: 381 (M⁺, 12 %), 163 (100).

### Beispiel 7 (Reaktionssequenz D)

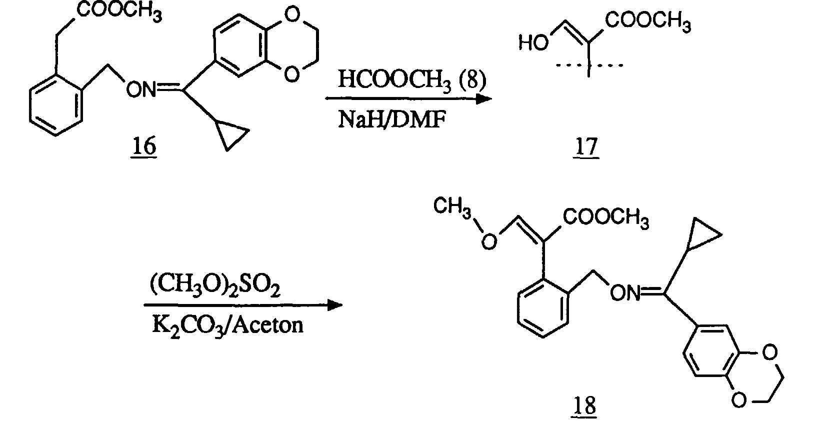

Eine Lösung von 39,8 g {[α-Cyclopropyl-3,4-ethylendioxybenzyl)imino]oxy}-o-tolylessigsäuremethylester 16 in 100 ml Dimethylformamid (DMF) und 62 ml Methylformiat wird langsam einer Suspension von 8,0 g Natriumhydrid in 300 ml Dimethylformamid zugetropft. Nach 3-stündigem Rühren des Reaktionsgemisches bei Raumtemperatur wird es mit Essigsäure angesäuert und danach mit Diethylether extrahiert. Die organische Phase wird zweimal mit Wasser und einmal mit Natriumchloridlösung gewaschen, getrocknet (Natriumsulfat) und im Vakuum eingeengt. Auf diese Weise erhält man als Rohprodukt den 2-[α-{[(α-Cyclopropyl-3,4-ethylendioxybenzyl)imino]oxy}-o-tolyl]-3-hydroxyacrylsäuremethylester 17, der ungereinigt weiter umgesetzt wird.

Das obige Ausgangsmaterial 17 wird zusammen mit 10,4 ml Dimethylsulfat, 15,2 g Kaliumcarbonat und 350 ml Aceton 2 Stunden bei Raumtemperatur gerührt. Dann wird filtriert, das Filtrat im Vakuum eingeengt und man erhält so ein rotbraunes Oel. Dieses wird an Kieselgel mit Tetrahydrofuran/n-Hexan (1:2) chromatographiert: 30 g [E]-2-[α-{[(α-Cyclopropyl-3,4-ethylendioxybenzyl)imino]oxy}-o-toly]-3-methoxy-acrylsäuremethylester 18 als gelbes Oel.

### Beispiel 8 (Reaktionssequenz C)

Herstellung von

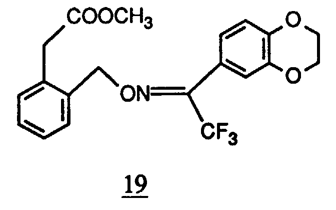

Wie im Beispiel 6 beschrieben, erhält man auf analoge Weise aus 50,0 g (1,4-Benzodioxan-6-yl)-trifluormethylketon und 49,8 g Aminhydrochlorid 4 in 420 ml Pyridin den Ester 19 als gelbes Oel.

### Beispiel 9 (Reaktionssequenz D)

Herstellung von

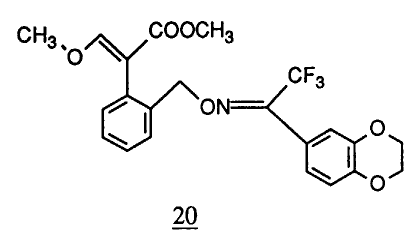

Das gemäss Beispiel 8 erhaltene Zwischenprodukt 19 wird analog zu Beispiel 7 mit Methylformiat zu einer Suspension von NaH in DMF getropft und so zum Hydroxyacrylsäuremethylester umgesetzt, welcher anschliessend mit Dimethylsulfat methyliert wird. Nach Chromatographie an Kieselgel mit Diethylether/n-Hexan (1:1) erhält man den 3-Methoxy-2-[α{[(α-trifluormethyl-3,4-ethylendioxybenzyl)imino]oxy}-o-tolyl]-acrylsäuremethylester 20 als hellgelbes Oel, MS: 451 (M⁺, 4 %), 145 (100).

### Beispiel 10 (Reaktionssequenz C)

Herstellung von

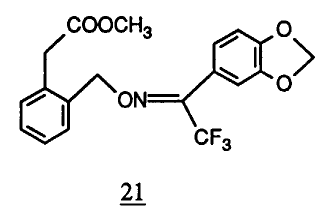

Gemäss Beispiel 6 erhält man analog aus 35,0 g (3,4-Methylendioxyphenyl)-trifluormethylketon und 37,5 g 4 den Ester 21 als gelbes Oel.

### Beispiel 11 (Reaktionssequenz D)

Herstellung von

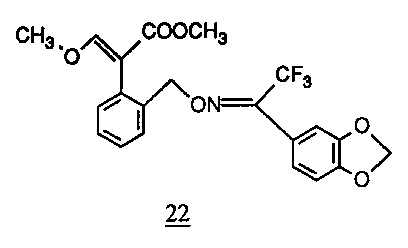

Analog zu Beispiel 7 erhält man aus dem Zwischenprodukt 21 mit Methylformiat und NaH in DMF den entsprechenden Hydroxyacrylsäuremethylester als Rohprodukt, welcher anschliessend mit Dimethylsulfat zu 22 methyliert wird. Chromatographie an Kieselgel mit Diethylether/n-Hexan (1:2) ergibt den 3-Methoxy-2-[α{[(α-trifluormethyl-3,4-methylendioxybenzyl)imino]oxy}-o-tolyl]acrylsäuremethylester als gelbes Oel, MS: 437 (M⁺,1%), 145(100).

### Beispiel 12 (Reaktionssequenz C)

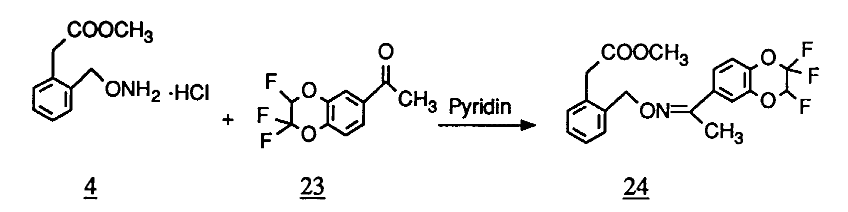

Gemäss Beispiel 6 erhält man analog aus 80,0 g des Ketons 23 und 79,7 g Aminhydrochlorid 4 den Ester 24.

### Beispiel 13 (Reaktionssequenz D)

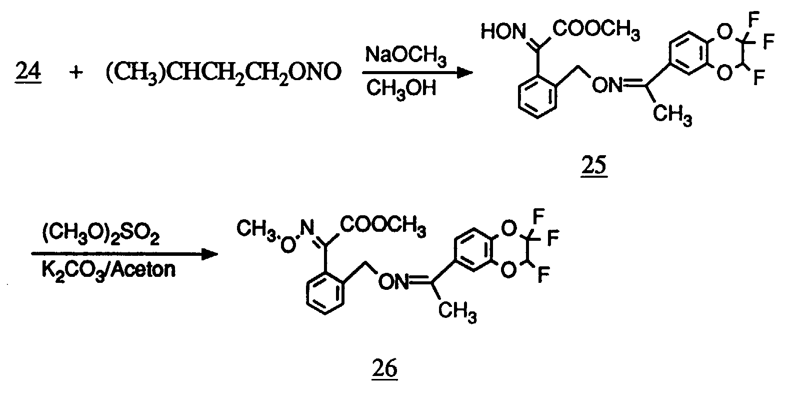

Eine Lösung von 50,0 g des gemäss Beispiel 12 erhaltenen Esters 24 und 24,0 g Isopentylnitrit in 100 ml Methanol wird langsam zu 34 ml Natriummethylat (30 % in Methanol) getropft. Nach 2 Stunden wird wie in Beispiel 5 beschrieben aufgearbeitet. Das so erhaltene Oxim 25 wird anschliessend mit Dimethylsulfat/Kaliumcarbonat in Aceton methyliert. Nach Chromatographie an Kieselgel (Diethylether/n-Hexan = 1:9) und Umkristallisation aus Diethylether/n-Hexan erhält man 26 als farblose Kristalle, Smp. 130-132°C.

### Beispiel 14 (Reaktionssequenz C)

Herstellung von

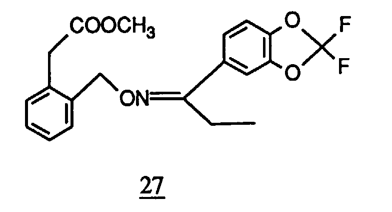

Gemäss Beispiel 6 erhält man analog aus 58,0 g 3,4-Difluormethylendioxypropiophenon und 62,7 g 4 den Ester 27 als gelbes Oel.

### Beispiel 15 (Reaktionssequenz D)

Herstellung von

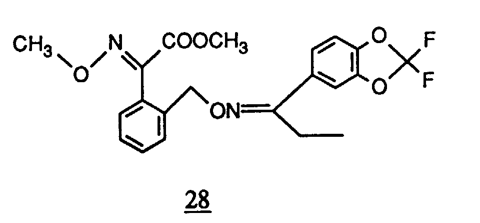

Aus 27 erhält man in Analogie zu Beispiel 13 mit Isopentylnitrit in Gegenwart von Na-Methylat in Methanol das entsprechende Oxim, welches weiter mit Dimethylsulfat in Gegenwart von K₂CO₃/Aceton zum Dioximether 28 umgesetzt wird. Nach Chromatographie an Kieselgel mit Ethylacetat/n-Hexan (1:9) kristallisiert 28 aus, Smp. 52-55°C.

### Beispiel 16 (Reaktionssequenz C)

Herstellung von

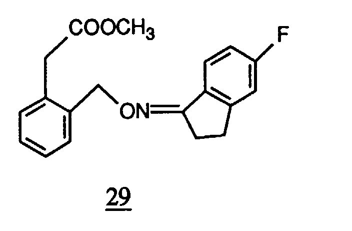

Gemäss Beispiel 6 erhält man analog aus 70,9 g Aminhydrochlorid 4 und 46,0 g 5-Fluorindan-1-on den Ester 29.

### Beispiel 17 (Reaktionssequenz D)

Herstellung von

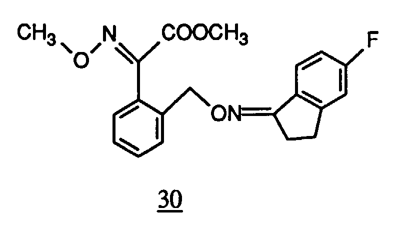

Aus dem gemäss Beispiel 16 erhaltenen Zwischenprodukt 29 erhält man in Analogie zu Beispiel 13 mit einem Nitrit, z.B. tert. Butylnitrit, das entsprechende Oxim, welches anschliessend methyliert wird. Nach Chromatographie an Kieselgel mit Dichlormethan/n-Hexan (9:1) erhält man 30 als farblose Kristalle, Smp. 89-92°C.

## Patentansprüche

1. 2-Aminooxymethylenphenylessigsäureester-Derivat der Formel I 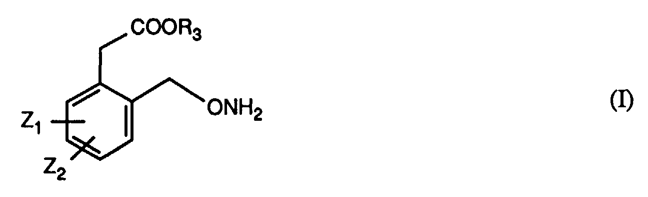 als freie Base und als Salz mit Proton-Säuren,
worin bedeuten
Z₁, Z₂ Wasserstoff, Halogen, Hydroxy, C₁-C₄-Alkyl, C₁-C₄-Alkoxy, C₁-C₄-Halogenalkyl, C₁-C₄-Halogenalkoxy, C₁-C₄-Alkylcarbonyl, C₁-C₄-Alkoxycarbonyl, Phenoxy, Nitro, Cyano,
Z₁ und Z₂ zusammen mit dem Phenylrest, an den sie gebunden sind, ein gegebenenfalls hydriertes Naphthalin, und
R₃ Wasserstoff oder C₁-C₁₂-Alkyl.

2. Verbindungen gemäss Anspruch 1, worin R₃ Wasserstoff oder C₁-C₄-Alkyl bedeutet.

3. Verbindungen gemäss Anspruch 2, worin R₃ Methyl bedeutet.

4. Verfahren zur Herstellung von Verbindungen der Formel I gemäß Anspruch 1, dadurch gekennzeichnet, dass man in Anwesenheit oder Abwesenheit eines Verdünnungsmittels in einem ersten Reaktionsschritt A einen substituierten o-Tolylessigsäureester II mit einem Benzoylhydroxylamin III zur Reaktion bringt 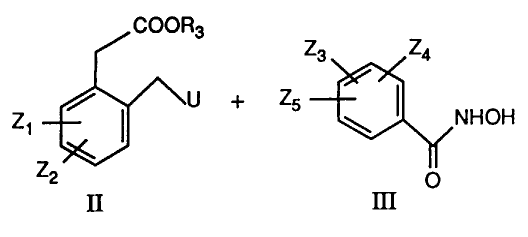 und den so gewonnenen benzoylierten 2-Aminooxymethylenphenylessigsäureester IV 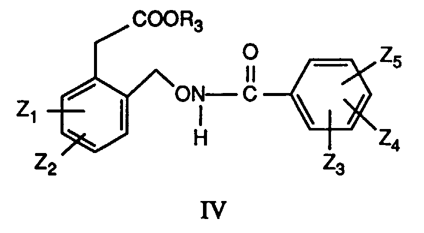 in einem zweiten Reaktionsschritt B einer sauren Verseifüng mit einer Säure HY unterwirft,
wobei R₃, Z₁ und Z₂ die für Formel I gegebene Bedeutung haben, während
Z₃, Z₄ und Z₅ gleich oder verschieden sind und Wasserstoff, gegebenenfalls verzweigtes C₁-C₆-Alkyl, Halogen, Hydroxy, gegebenenfalls verzweigtes C₁-C₆-Alkoxy, Nitro, Cyano, N(R₅)₂, SO₂NH₂, gegebenenfalls substituiertes Phenoxy, CF₃, Mercapto bedeuten, wobei die beiden
R₅ gleich oder verschieden sind und Wasserstoff und/oder gegebenenfalls verzweigtes C₁-C₆-Alkyl bedeuten,
und worin auch
Z₃ und Z₄ in benachbarter Stellung zusammen eine Methylendioxy-Brücke bilden oder mit dem Phenylring, an den sie gebunden sind, einen Naphthylring bilden können, und worin U eine nucleofuge Abgangsgruppe bedeutet.

5. Verfahren gemäss Anspruch 4, wobei man beide Teilschritte in Gegenwart eines Verdünnungsmittels durchführt.

6. Verfahren gemäss Anspruch 4, worin Z₃, Z₄ und Z₅ unabhängig voneinander Wasserstoff, Methyl und Halogen sind.

7. Verfahren gemäss Anspruch 6, worin Z₃=Z₄=Z₅=Wasserstoff bedeuten.

8. Verfahren gemäss Anspruch 4, worin
U Halogenid (wie Chlorid, Bromid, Jodid), Benzolsulfonat, Toluolsulfonat, Methansulfonat, Triflat oder Acetat bedeutet.

9. Verfahren gemäss Anspruch 8, worin U Bromid bedeutet.

10. Verfahren gemäss Anspruch 4, wobei man den Teilschritt A in Gegenwart einer Base durchführt.

11. Verfahren gemäss Anspruch 10, worin die verwendete Base Natriumcarbonat oder Kaliumcarbonat ist.

12. Verfahren gemäss Anspruch 5, worin das im Reaktionsschritt A verwendete Verdünnungsmittel ein Ether, ein Keton, ein Amid, ein Kohlenwasserstoff, ein halogenierter Kohlenwasserstoff, ein Nitril, Dimethylsulfoxid oder ein Ester ist.

13. Verfahren gemäss Anspruch 12, worin das verwendete Verdünnungsmittel Acetonitril ist.

14. Verfahren gemäss Anspruch 4, worin die Reaktionstemperatur im Schritt A zwischen 0°C und der Siedetemperatur des Verdünnungsmittels liegt.

15. Verfahren gemäss Anspruch 14, worin die Reaktionstemperatur zwischen 60°C und 65°C liegt.

16. Verfahren gemäss Anspruch 4, worin HY Halogenwasserstoffsäure (wie Chlorwasserstoff-, Bromwasserstoff-, Jodwasserstoffsäure), Sulfonsäure (wie gegebenenfalls substituierte Benzolsulfonsäure, Methansulfonsäure, Trifluormethansulfonsäure), Carbonsäure (wie Ameisensäure, Essigsäure, Trifluoressigsäure), Perchlorsäure, Tetrafluorborsäure, Phosphorsäure, Hexafluorphosphorsäure, Schwefelsäure oder schweflige Säure bedeutet.

17. Verfahren gemäss Anspruch 16, worin HY Chlorwasserstoff oder Schwefelsäure bedeutet.

18. Verfahren gemäss Anspruch 5, worin das im Reaktionsschritt B verwendete Verdünnungsmittel ein Alkohol der Formel R₃-OH ist.

19. Verfahren gemäss Anspruch 4, worin die im Reaktionsschritt B angewendete Temperatur zwischen 0°C und der Siedetemperatur des Verdünnungsmittels liegt.

20. Verfahren gemäss Anspruch 19, worin die Reaktionstemperatur zwischen 20°C und der Siedetemperatur des Verdünnungsmittels liegt.

21. Verwendung von Verbindungen der Formel I gemäß Anspruch 1 zur Gewinnung von Verbindungen der Formel VI 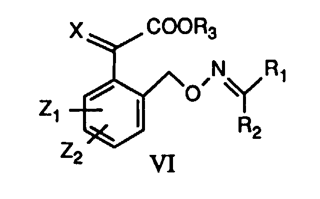 worin bedeuten
X CHO(C₁-C₄-Alkyl), NO(C₁-C₄-Alkyl), oder CH₂;
R₁ und R₂ gleich oder verschieden Wasserstoff, Cyano, gegebenenfalls verzweigtes C₁-C₁₀-Alkyl, C₁-C₄-Halogenalkyl, C₃-C₆-Cycloalkyl, C₃-C₆-Halogencycloalkyl, C₃-C₆-Cycloalkyl-C₁-C₄-alkyl, C₁-C₄-Alkoxy-C₁-C₄-alkyl, C₁-C₄-Alkylthio-C₁-C₄-alkyl, Arylthio-C₁-C₄-alkyl, C₂-₆-Alkenyl, C₂-C₅-Halogenalkenyl, C₃-C₆-Cycloalkenyl, C₃-C₆-Halogencycloalkenyl, C₂-C₆-Alkinyl, C₁-C₆-Alkoxy, C₁-C₆-Halogenalkoxy, C₁-C₄-Alkylthio, Benzylthio, C₁-C₄-Alkylcarbonyl, gegebenenfalls substituiertes Phenylcarbonyl, gegebenenfalls substituiertes Benzylcarbonyl, C₁-C₄-Alkoxycarbonyl, gegebenenfalls substituiertes Phenoxycarbonyl, gegebenenfalls substituiertes Benzyloxycarbonyl, gegebenenfalls substituiertes Aryl, gegebenenfalls substituiertes Aryloxy, gegebenenfalls substituiertes Arylthio, gegebenenfalls substituiertes Aryl-C₁-C₄-alkyl, gegebenenfalls substituiertes Aryl-C₁-C₄-alkenyl, gegebenenfalls substituiertes Aryloxy-C₁-C₄-alkyl, gegebenenfalls substituiertes Arylthio-C₁-C₄-alkyl, gegebenenfalls substituiertes Heteroaryl, gegebenenfalls substituiertes Heteroaryloxy, gegebenenfalls substituiertes Heteroarylthio, gegebenenfalls substituiertes Heteroaryl-C₁-C₄-alkyl, gegebenenfalls substituiertes Heteroaryl-C₂-C₄-alkenyl, gegebenenfalls substituiertes Heteroaryloxy-C₁-C₄-alkyl, gegebenenfalls substituiertes Heterocyclyl, gegebenenfalls substituiertes Heterocyclyloxy, gegebenenfalls substituiertes Heterocyclylalkyl, N(R₄)₂, wobei die Bedeutungen von R₄ gleich oder verschieden sind und H, C₁-C₆-Alkyl, gegebenenfalls substituiertes Phenyl bedeuten, oder beide R₄, zusammen mit dem Stickstoffatom, an das sie gebunden sind, einen 5- bis 7-gliedrigen, gegebenenfalls substituierten Ring bilden, der Heteroatome aufweisen kann; oder R₁ und R₂ -CON(R₄)₂, Nitro, Halogen, -S(O)ₙR₄ bedeuten, worin n 0, 1 oder 2, ist oder (CH₂)ₘPO(OR₄)₂, worin m 0 oder 1 ist, oder R₁ und R₂ zusammen einen carbocyclischen oder heterocyclischen Ring bilden, der gegebenenfalls substituiert sein kann,
während R₃, Z₁ und Z₂ die für Formel I genannte Bedeutung haben.

22. Verfahren zur Herstellung von Verbindungen der Formel VI durch
A) Reaktion eines substituierten o-Tolylessigsäureesters der Formel II 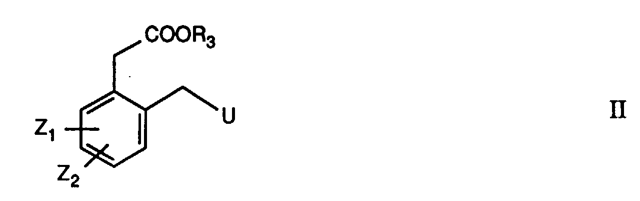 mit einem Benzoylhydroxylamin III 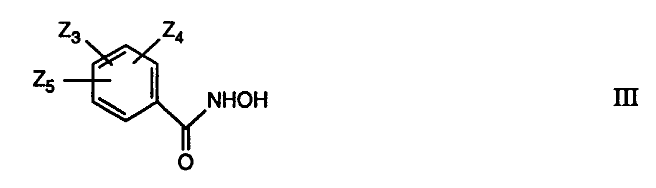
B) saure Verseifung des so gewonnenen benzoylierten 2-Aminooxymethylenphenylessigsäureesters der Formel IV 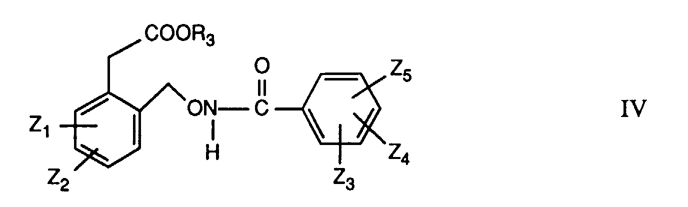 mit einer Säure HY,
C) Reaktion des 2-Aminooxymethylenphenylessigsäureesters der Formel I, gemäß Anspruch 1 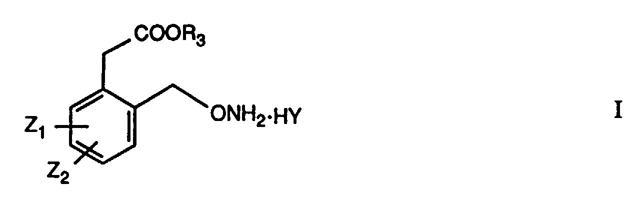 mit einem Keton R₁-CO-R₂ und
D) Ueberführung der Acetat-Seitenkette des gebildeten Zwischenprodukts V 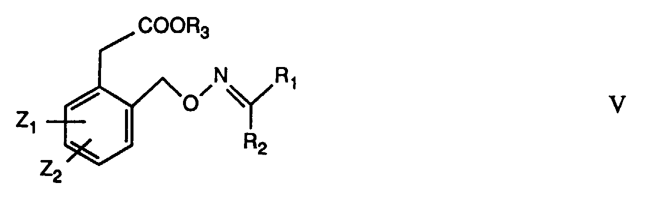 in die Seitenkette 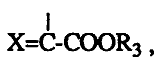 worin X wahlweise C₁-C₄-Alkyl-O-CH oder C₁-C₄-Alkyl-O-N bedeutet, wobei U, R₃ und Z₁ bis Z₅ die im Anspruch 4 gegebene Bedeutung haben, R₁ und R₂ die Bedeutung gemäss Anspruch 21 haben, während Y ein Säureanion ist.

## Claims

1. A 2-aminooxymethylenephenylacetic ester derivative of formula I 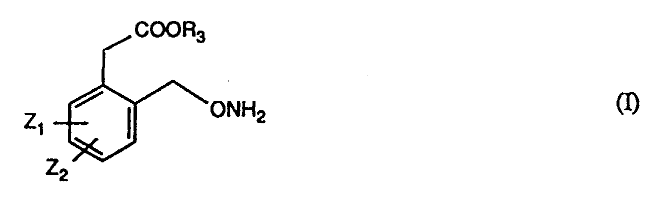 as free base and as salt with protic acids,
wherein
Z₁ and Z₂ are hydrogen, halogen, hydroxyl, C₁-C₄alkyl, C₁-C₄alkoxy, C₁-C₄-haloalkyl, C₁-C₄haloalkoxy,
C₁-C₄alkylcarbonyl, C₁-C₄alkoxycarbonyl, phenoxy, nitro, cyano,
Z₁ and Z₂, together with the phenyl radical to which they are attached, are naphthalene or hydrogenated naphthalene, and R₃ is hydrogen or C₁-C₁₂alkyl.

2. A compound according to claim 1, wherein R₃ is hydrogen or C₁-C₄alkyl.

3. A compound according to claim 1, wherein R₃ is methyl.

4. A process for the preparation of a compound of formula I according to claim 1, which comprises, in a first reaction step A, reacting a substituted o-tolylacetate II with a benzoyl hydroxylamine III 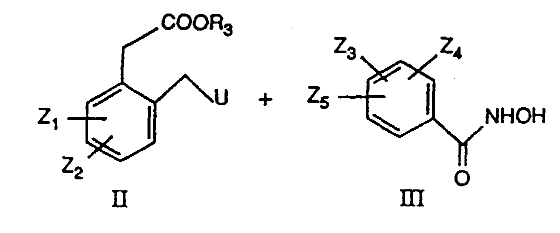 in the absence or presence of a diluent, and subjecting the benzoylated 2-aminooxymethylenephenylacetate IV thus obtained 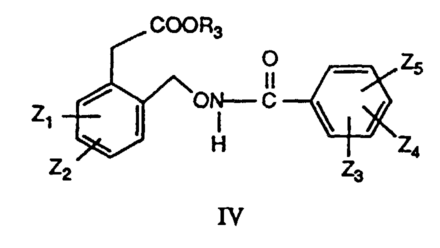 in a second reaction step B to acid saponification with an acid HY, where R₃, Z₁ and Z₂ are as defined for formula I, while
Z₃, Z₄ and Z₅ are identical or different and are hydrogen, unbranched or branched C₁-C₆alkyl, halogen, hydroxyl, unbranched or branched C₁-C₆alkoxy, nitro, cyano, N(R₅)₂, SO₂NH₂, unsubstituted or substituted phenoxy, CF₃, mercapto, and the two substituents
R₅ are identical or different and are hydrogen and/or unbranched or branched C₁-C₆alkyl,
and wherein also
Z₃ and Z₄ in vicinal position together form a methylenedioxy bridge or, together with the phenyl ring to which they are attached, can form a naphthyl ring, and wherein U is a nucleofugic leaving group.

5. A process according to claim 4, wherein both partial steps are carried out in the presence of a diluent.

6. A process according to claim 4, wherein Z₃, Z₄ and Z₅ are each independently of one another hydrogen, methyl and halogen.

7. A process according to claim 6, wherein Z₃=Z₄=Z₅ and are hydrogen.

8. A process according to claim 4, wherein
U is halide (such as chloride, bromide or iodide), benzenesulfonate, toluenesulfonate, methanesulfonate, triflate or acetate.

9. A process according to claim 8, wherein U is bromide.

10. A process according to claim 4, wherein the partial step A is carried out in the presence of a base.

11. A process according to claim 10, wherein the base used is sodium carbonate or potassium carbonate.

12. A process according to claim 5, wherein the diluent used in reaction step A is an ether, a ketone, an amide, a hydrocarbon, a halogenated hydrocarbon, a nitrile, dimethyl sulfoxide or an ester.

13. A process according to claim 12, wherein the diluent used is acetonitrile.

14. A process according to claim 4, wherein the reaction temperature in step A is between 0°C and the boiling temperature of the diluent.

15. A process according to claim 14, wherein the reaction temperature is between 60°C and 65°C.

16. A process according to claim 4, wherein HY is hydrohalic acid (such as hydrochloric, hydrobromic or hydriodic acid), sulfonic acid (such as unsubstituted or substituted benzenesulfonic acid, methanesulfonic acid, trifluoromethanesulfonic acid), carboxylic acid (such as formic acid, acetic acid, trifluoroacetic acid), perchloric acid, tetrafluoroboric acid, phosphoric acid, hexafluorophosphoric acid, sulfuric acid or sulfurous acid.

17. A process according to claim 16, wherein HY is hydrogen chloride or sulfuric acid.

18. A process according to claim 5, wherein the diluent used in reaction step B is an alcohol of formula R₃-OH.

19. A process according to claim 4, wherein the temperature employed in reaction step B is between 0°C and the boiling temperature of the diluent.

20. A process according to claim 19, wherein the reaction temperature is between 20°C and the boiling temperature of the diluent.

21. Use of a compound of formula I according to claim 1 to obtain a compound of formula VI 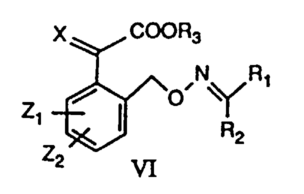 wherein
X is CHO(C₁-C₄alkyl), NO(C₁-C₄alkyl), or CH₂;
R₁ and R₂ may be identical or different and are hydrogen, cyano, unbranched or branched C₁-C₁₀alkyl, C₁-C₄haloalkyl, C₃-C₆cycloalkyl, C₃-C₆halocycloalkyl, C₃-C₆cycloalkyl-C₁-C₄alkyl, C₁-C₄alkoxy-C₁-C₄alkyl, C₁-C₄alkylthio-C₁-C₄alkyl, arylthio-C₁-C₄alkyl, C₂-C₆alkenyl, C₂-C₅haloalkenyl, C₃-C₆cycloalkenyl, C₃-C₆halocycloalkenyl, C₂-C₆alkynyl, C₁-C₆alkoxy, C₁-C₆haloalkoxy, C₁-C₄alkylthio, benzylthio, C₁-C₄alkylcarbonyl, unsubstituted or substituted phenylcarbonyl, unsubstituted or substituted benzyl carbonyl, C₁-C₄alkoxycarbonyl, unsubstituted or substituted phenoxycarbonyl, unsubstituted or substituted benzyloxycarbonyl, unsubstituted or substituted aryl, unsubstituted or substituted aryloxy, unsubstituted or substituted arylthio, unsubstituted or substituted aryl-C₁-C₄alkyl, unsubstituted or substituted aryl-C₁-C₄alkenyl, unsubstituted or substituted aryloxy-C₁-C₄alkyl, unsubstituted or substituted arylthio-C₁-C₄alkyl, unsubstituted or substituted heteroaryl, unsubstituted or substituted heteroaryloxy, unsubstituted or substituted heteroarylthio, unsubstituted or substituted heteroaryl-C₁-C₄alkyl, unsubstituted or substituted heteroaryl-C₂-C₄alkenyl, unsubstituted or substituted heteroaryloxy-C₁-C₄alkyl, unsubstituted or substituted heterocyclyl, unsubstituted or substituted heterocyclyloxy, unsubstituted or substituted heterocyclylalkyl, N(R₄)₂, where the meanings of R₄ are identical or different and are H, C₁-C₆alkyl, unsubstituted or substituted phenyl, or both substituents R₄, together with the nitrogen atom to which they are attached, form a 5- to 7-membered unsubstituted or substituted ring that may contain heteroatoms; or R₁ and R₂ are -CON(R₄)₂. nitro, halogen, -S(O)ₙR₄, wherein n is 0, 1 or 2, or (CH₂)ₘPO(OR₄)₂, wherein m is 0 or 1, or R₁ and R₂ together form a carbocyclic or heterocyclic ring that may be unsubstituted or substituted,
while R₃, Z₁ and Z₂ are as defined for formula I.

22. A process for the preparation of a compound of formula VI, by
A) reacting a substituted o-tolylacetate of formula II 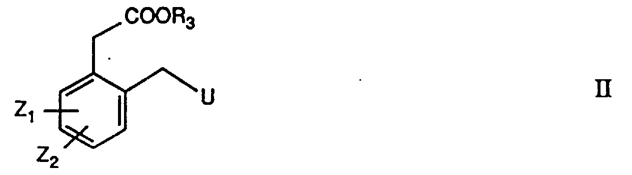 with a benzoyl hydroxylamine III 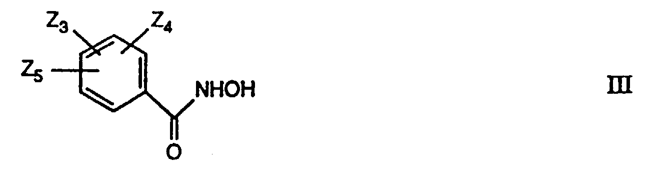
B) subjecting the benzoylated 2-aminooxymethylenephenylacetate thus obtained of formula IV 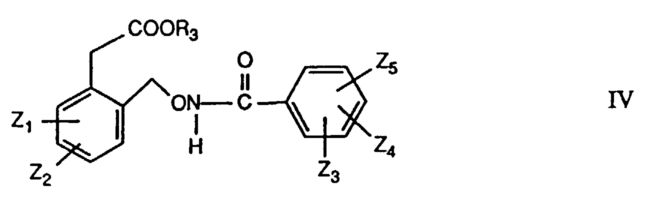 to acid saponification with an acid HY,
C) reacting the 2-aminooxymethylenephenylacetate of formula I according to claim 1 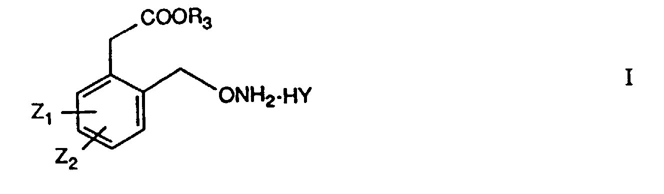 with a ketone R₁-CO-R₂, and
D) converting the acetate side-chain of the resultant intermediate V 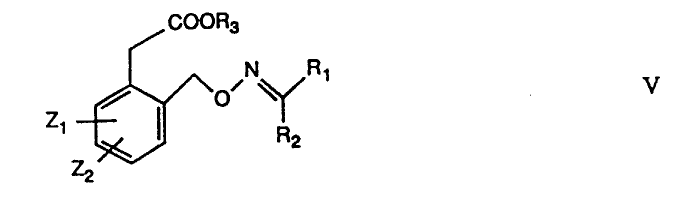 into the side-chain 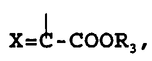 wherein X is alternatively C₁-C₄alkyl-O-CH or C₁-C₄alkyl-O-N, and U, R₃ and Z₁ to Z₅ are as defined in claim 4, R₁ and R₂ are as defined in claim 21, and Y is an acid anion.

## Revendications

1. Dérivé d'esters de l'acide 2-aminooxyméhylènephénylacétique de formule I 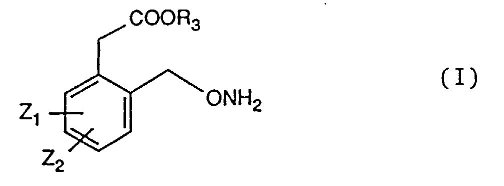 sous forme de base libre et sous forme de sel avec des acides protoniques,
où
Z₁ et Z₂ représentent l'hydrogène, un halogène, l'hydroxy, un alkyle en C₁-C₄, un alcoxy en C₁-C₄, un halogénoalkyle en C₁-C₄, un halogénoalcoxy en C₁-C₄, un alkyle en C₁-C₄ carbonyle, un alcoxy en C₁-C₄ carbonyle, le phénoxy, le nitro, le cyano,
Z₁ et Z₂ représentent ensemble avec le reste phényle auquel ils sont liés, un naphtalène éventuellement hydrogéné et
R₃ représente l'hydrogène ou un alkyle en C₁-C₁₂.

2. Composés selon la revendication 1, où R₃ représente l'hydrogène ou un alkyle en C₁-C₄.

3. Composés selon la revendication 2, où R₃ représente le méthyle.

4. Procédé pour la préparation des composés de formule I selon la revendication 1, caractérisé en ce que l'on fait. réagir, dans une première étape réactionnelle A, en présence ou non d'un diluant, un ester de l'acide o-tolylacétique II substitué avec une benzoylhydroxylamine III 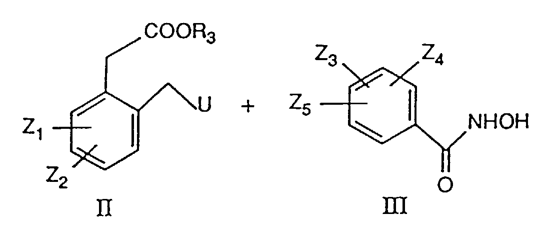 et en ce que l'on soumet, dans une seconde étape réactionnelle B, l'ester de l'acide 2-aminooxyméthylènephénylacétique IV benzoylé ainsi obtenu 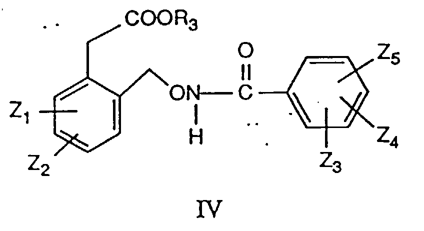 à une saponification acide avec un acide HY,
où R₃, Z₁ et Z₂ ont les significations données pour la formule I, tandis que
Z₃, Z₄ et Z₅ sont identiques ou différents et représentent l'hydrogène, un alkyle en C₁-C₆ éventuellement ramifié, un halogène, l'hydroxy, un alcoxy en C₁-C₆ éventuellement. ramifié, le nitro, le cyano, N(R₅)₂, SO₂NH₂, le phénoxy éventuellement substitué, CF₃, le mercapto, les deux R₅ étant identiques ou différents et représentant l'hydrogène et/ou un alkyle en C₁-C₆ éventuellement ramifié,
et où également.
Z₃ et Z₄ en position adjacente forment ensemble un pont méthylènedioxy ou peuvent former avec le noyau phénylique auquel ils sont liés, un noyau naphtyle, et où U représente un groupe partant nucléofuge.

5. Procédé selon la revendication 4, dans lequel on exécute les deux étapes en présence d'un diluant..

6. Procédé selon la revendication 4, où Z₃, Z₄ et Z₅ sont, indépendamment les uns des autres, l'hydrogène, le méthyle et un halogène.

7. Procédé selon la revendication 6, où R₃, Z₄ et Z₅ représentent l'hydrogène.

8. Procédé selon la revendication 4, où U représente un halogénure (tel que le chlorure, le bromure, l'iodure), le benzènesulfonate, le toluènesulfonate, le méthanesulfonate, le triflate ou l'acétate.

9. Procédé selon la revendication 8, où U représente le bromure.

10. Procédé selon la revendication 4, dans lequel on exécute l'étape A en présence d'une base.

11. Procédé selon la revendication 10, dans lequel la base utilisée est le carbonate de sodium ou le carbonate de potassium.

12. Procédé selon la revendication 5, dans lequel le diluant utilisé dans l'étape réactionnelle A est un éther, une cétone, un amide, un hydrocarbure, un hydrocarbure halogéné, un nitrile, le diméthylsulfoxyde ou un ester.

13. Procédé selon la revendication 12, dans lequel le diluant utilisé est l'acétonitrile.

14. Procédé selon la revendication 4, dans lequel la température réactionnelle dans l'étape A est comprise entre 0°C et la température d'ébullition du diluant.

15. Procédé selon la revendication 14, dans lequel la température réactionnelle est comprise entre 60°C et 65°C.

16. Procédé selon la revendication 4, où HY représente un hydracide halogéné (tel que les acides chlorhydrique, bromhydrique et iodhydrique), un acide sulfonique (tel que les acides benzènesulfonique, méthanesulfonique, trifluorométhanesulfonique éventuellement substitués), un acide carboxylique (tel que les acides formique, acétique, trifluoroacétique), l'acide perchlorique, l'acide tétrafluoroborique, l'acide phosphorique, l'acide hexafluorophosphorique, l'acide sulfurique ou l'acide sulfureux.

17. Procédé selon la revendication 16, où HY représentée l'acide chlorhydrique ou l'acide sulfurique.

18. Procédé selon la revendication 5, dans lequel le diluant utilisé dans l'étape réactionnelle B est un alcool de formule R₃-OH.

19. Procédé selon la revendication 4, dans lequel la température mise en oeuvre dans l'étape réactionnelle B est comprise entre 0°C et. la température d'ébullition du diluant.

20. Procédé selon la revendication 19, dans lequel la température réactionnelle est comprise entre 20°C et la température d'ébullition du diluant.

21. Utilisation des composés de formule I selon la revendication 1 pour la préparation des composés de formule VI 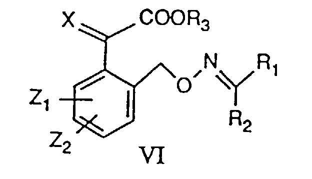 où
X représente CHO(alkyle en C₁-C₄), NO(alkyle en C₁-C₄) ou CH₂;
R₁ et R₂ sont identiques ou différents et représentent l'hydrogène, le cyano, un alkyle en C₁-C₁₀ éventuellement ramifié, un halogénoalkyle en C₁-C₄, un cycloalkyle en C₃-C₆, un halogénocycloalkyle en C₃-C₆, un cycloalkyle en C₃-C₆ alkyle en C₁-C₄, un alcoxy en C₁-C₄ alkyle en C₁-C₄, un alkyle en C₁-C₄ thioalkyle en C₁-C₄, un arylthioalkyle en C₁-C₄, un alcényle en C₂-C₆, un halogénoalcényle en C₂-C₅, un cycloalcényle en C₃-C₆, un halogénocycloalcényle en C₃-C₆, un alcynyle en C₂-C₆, un alcoxy en C₁-C₆, un halogénoalcoxy en C₁-C₆, un alkyle en C₁-C₄ thio, le benzylthio, un alkyle en C₁-C₄ carbonyle, un phénylcarbonyle éventuellement substitué, un benzylcarbonyle éventuellement substitué, un alcoxy en C₁-C₄ carbonyle, un phénoxycarbonyle éventuellement substitué, un benzyloxycarbonyle éventuellement substitué, un aryle éventuellement substitué, un aryloxy éventuellement substitué, un arylthio éventuellement substitué, un arylalkyle en C₁-C₄ éventuellement substitué, un arylalcényle en C₁-C₄ éventuellement substitué, un aryloxyalkyle en C₁-C₄ éventuellement substitué, un arylthioalkyle en C₁-C₄ éventuellement substitué, un hétéroaryle éventuellement substitué, un hétéroaryloxy éventuellement substitué, un hétéroarylthio éventuellement substitué, un hétéroarylalkyle en C₁-C₄ éventuellement substitué, un hétéroarylalcényle en C₂-C₄ éventuellement substitué, un hétéroaryloxyalkyle en C₁-C₄ éventuellement substitué, un hétérocyclyle éventuellement substitué, un hétérocyclyloxy éventuellement substitué, un hétérocyclylalkyle éventuellement substitué, N(R₄)₂, les significations de R₄ étant identiques ou différentes et représentant H, un alkyle en C₁-C₆, un phényle éventuellement substitué ou les deux R₄ formant ensemble avec l'atome d'azote auquel ils sont liés, un noyau ayant 5 à 7 chaînons, éventuellement substitué, pouvant présenter des hétéroatomes; ou R₁ et R₂ représentent - CON(R₄)₂, le nitro, un halogène, -S(O)ₙR₄, n étant égal à 0, 1 ou 2 ou (CH₂)ₘPO(OR₄)₂, m étant égal à 0 ou 1; ou R₁ et R₂ forment ensemble un noyau carbocyclique ou hétérocyclique, pouvant être éventuellement substitué, tandis que R₃, Z₁ et Z₂ ont les significations indiquées pour la formule I.

22. Procédé pour la préparation des composés de formule VI, caractérisé par
A) la réaction d'un ester de l'acide o-tolylacétique substitué de formule II 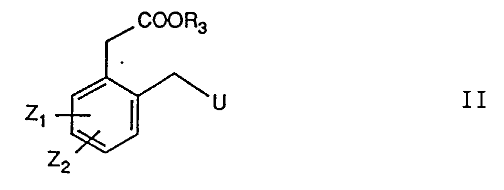 avec une benzoylhydroxylamine III 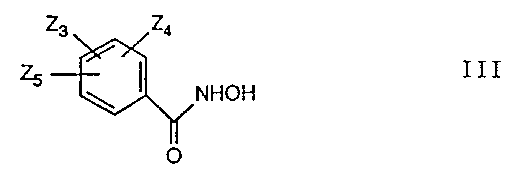
B) la saponification acide de l'ester de l'acide 2-aminooxyméthylènephénylacétique benzoylé de formule IV ainsi obtenu 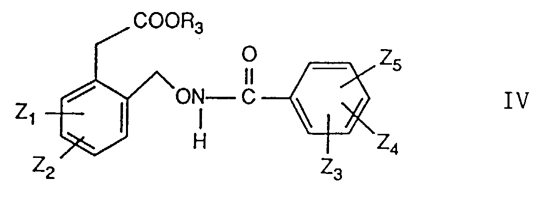 avec un acide HY,
C) la réaction de l'ester de l'acide 2-aminooxyméthylènephénylacétique de formule I selon la revendication 1 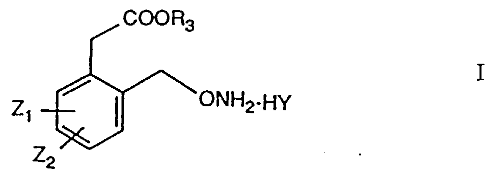 avec une cétone R₁-CO-R₂ et
D) la transformation de la chaîne latérale acétate du produit intermédiaire V formé 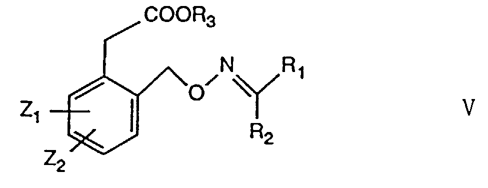 en une chaîne latérale 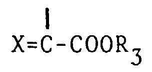 où X représente, selon les nécessités, un alkyle en C₁-C₄-O-CH ou un alkyle en C₁-C₄-O-N, U, R₃ et Z₁ à Z₅ ayant les significations données dans la revendication 4, R₁ et R₂ ayant les significations selon la revendication 21, tandis que Y est. un anion d'acide.
